# EUROPEAN PATENT APPLICATION

(11) **EP 2 070 527 A1**
(43) Date of publication of application: **17.06.2009**
(21) Application number: 07119226.4
(22) Date of filing: 24.10.2007
(51) Int. Cl.: A61K 31/167, A61K 31/40, A61K 31/42, A61K 31/421, A61K 31/495, A61P 31/10, A61P 27/02

(54) **Inhibitors of fungal biofilm-formation**

(71) Applicant: QuoNova Europe GmbH, 82152 München (DE)
(72) Inventor: Ammendola, Aldo, 80637, München (DE)
(74) Representative: Vossius & Partner

(57) **Abstract**

The present invention is directed to compounds for treating diseases associated with fungal infection by a fungal species capable of forming biofilms, as well as to the use of compounds to remove or diminish fungal infestation in a non-therapeutic context.

## Description

### BACKGROUND

Extracellular autoinducing compounds in the supernatants of microbial cultures were first recognized for their roles in the induction of genetic competence in gram-positive bacteria and in the regulation of light production in marine *Vibrio* species. "Quorum sensing" enables bacterial cells to chemically measure the density of the surrounding population. Subsequently, many examples of cell density-dependent gene regulation by extracellular signal molecules have been found in diverse microorganisms. The widespread incidence of diverse quorum-sensing systems strongly suggests that regulation in accordance with cell density is important for the success of microbes in many environments. Cell density-dependent regulatory networks in microorganisms generally control processes that involve cell-cell interactions, such as group motility and the formation of multicellular structures. In a wide array of environmental and medically relevant bacteria, the development, maintenance, and dispersion of multicellular, surface-associated biofilms are in part controlled by quorum-sensing regulatory pathways. The uptake of extracellular DNA is often regulated in accordance with cell density presumably to enhance the chances of taking up DNA from closely related strains. For some bacteria, a link between the competence and biofilm formation has been established. Because quorum sensing has been implicated as an important factor in the expression of virulence genes in animal and plant pathogens, including the formation of biofilms, quorum sensing blocking agents show promise for a novel antifouling and anti-microbial approach.

Biofilms are defined as an association of microorganisms growing attached to a surface and producing a slime layer of extracellular polymers in which the microbial consortia is embedded in a protective environment. Biofilms represent a severe problem as, for instance, bacteria integrated in such a polymer matrix develop resistance to conventional antimicrobial agents. *P. aeruginosa* cells, for example, growing in an alginate slime matrix have been demonstrated to be resistant to antibiotics (e. g., aminoglycosides, P-lactam antibiotics, fluoroquinolones) and disinfectants (Govan & Deretic, Microbiol. Rev. 60: 539-74,1996).

Recently, it has become apparent that fungi, like bacteria, also use quorum regulation to affect population-level behaviors such as biofilm formation and pathogenesis. Considering the extent to which quorum sensing regulation controls important processes in many distantly related bacterial genera, it is not surprising that cell density-dependent regulation also appears to be prevalent in diverse fungal species. A review on the details of quorum sensing regulation in *Candida albicans* and other fungi that appear to use quorum sensing regulation can be found in: D.A. Hogan: Talking to Themselves - Autoregulation and Quorum Sensing in Fungi, Eukaryotic Cell, 2006, Vol. 5, No. 4, p. 613-619.

The discovery that a wide spectrum of organisms use quorum sensing to control virulence factor production and other phenotypes such as biofilm formation makes it an attractive target for antimicrobial therapy. Pathogenic organisms using this signaling system to control virulence could potentially be rendered avirulent by blocking this cell-cell communication system. In contrast to traditional antibiotics, the risk of resistance development seems to be very low, since quorum sensing blocking agents would not kill the organism but disturb signal transduction pathways. There are several possibilities of interrupting the quorum sensing circuit.

The predominant quorum sensing system used by many Gram-negative bacteria is the homoserine-lactone (HSL) based signaling system. Different pathways to interrupt this quorum sensing system have been reported. For example, plants expressing an HSL-lactonase enzyme originally derived from *Bacillus sp*. have been demonstrated to quench pathogen quorum sensing signaling and to significantly enhance resistance to *Erwinia carotovora* infections (Dong et al., Nature 411: 813-7, 2001). An alternative way to block cell signaling could be to interrupt the HSL synthesis by using analogs of HSL precursors.

However, the most promising possibility to block quorum sensing is to take advantage of the unique specificity the HSLs and HSL-receptor proteins show for one another. The ability of homoserine lactone-based analogs to inhibit activation of HSL-receptor proteins has already been demonstrated in a number of bacteria including *Vibrio fischeri* (Schaefer et al., J. Bacteriol. 178: 2897-901, 1996), *Agrobacterium tumefaciens* (Zhu et al., J. Bacteriol. 180: 5398-405, 1998), *Chromobacterium violaceum* (McLean et al., Microbiology 143: 3703- 11, 1997), *Aeromonas salmonicida* (Swift et al., J. Bacteriol. 179: 5271-81, 1997) and *Pseudomonas aeruginosa* (Pesci et al., J. Bacteriol. 179: 3127-32, 1997). However, none of these compounds have been developed as antimicrobial agents, e. g. in medical therapy, so far.

The non-HSL-based antimicrobials in the prior art which are supposed to interfere specifically with HSL-regulated processes mainly are halogenated furanone derivatives which are structurally similar to HSLs and have been isolated from red marine algae *Delisea pulchra* (WO 96/29392). Additionally, these substances have been demonstrated to inhibit also Gram-positive bacteria (WO 99/53915). However, the use of most of these compounds is limited due to their toxicity making them unsuitable for veterinary and medical applications.

Many target genes involved in biofilm formation, methods of screening for compounds to control biofilm development and HSL-based compositions to prevent biofilm formation have been described (WO 99/55368, WO 98/57618, WO 99/27786, WO 98/58075). EP 1 478 364 discloses the use of specific non-furanone-based compounds for the regulation of the quorum sensing system of microorganisms, whereby the focus lies on antibacterial applications encompassing such compounds.

The aim of the present invention is to provide compounds for use against fungal microorganisms which are capable of forming biofilms.

### DETAILED DESCRIPTION

An embodiment of the present invention are compounds according to Formula I for treating diseases associated with fungal infection by a fungal species capable of forming biofilms, 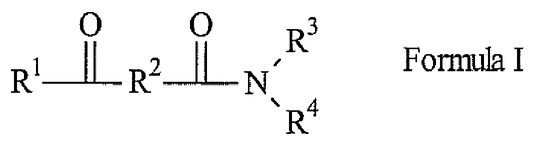 wherein

| | | |
|---|---|---|
| R¹ | represents | C₁-C₂₀-alkylene, or |
| | | C₁-C₂₀-alkenylene, or |
| | | C₁-C₂₀-alkynylene, |

which may contain one or more group(s) Z;
- Z: is selected from the group comprising S, O, N, NR₄, CO, CO₂, CS, SO or SO₂;

| | | |
|---|---|---|
| R² | represents | C₁-C₂₀-alkylene, or |
| | | C₁-C₂₀-alkenylene, or |
| | | C₁-C₂₀-alkynylene, |

- R³ and R⁴: taken together may form a 5-membered or 6-membered aromatic or non-aromatic ring which may contain one or more heteroatoms, wherein the heteroatoms may be independently of each other O or N;
wherein the ring may carry one or more substituents Y, or alternatively
- R³: may be H or CH₃ and
- R⁴: may be a 5-membered or 6-membered aromatic or non-aromatic ring which may contain one or more heteroatoms, wherein the heteroatoms may be independently of each other O or N;
wherein the ring may carry one or more substituents Y; and
- Y: is selected from the group comprising amine, alkylamine, C₁-C₃ alkyl, C₁-C₃ alkenyl, C₁-C₃ alkynyl, halogen, C₁-C₃ alkyl substituted by one or more halogens.

In a preferred embodiment of the present invention, the compounds for treating diseases associated with fungal infection are compounds according to Formula I, wherein
- R¹: represents C₆-C₂₀-alkylene, or C₁-C₂₀-alkenylene, or C₁-C₂₀-alkynylene,

In another preferred embodiment of the present invention, the compounds for treating diseases associated with fungal infection by a fungal species capable of forming biofilms are compounds according to Formula I, wherein
- R¹: represents C₆-C₂₀-alkylene.

In another preferred embodiment of the present invention, the compounds for treating diseases associated with fungal infection by a fungal species capable of forming biofilms are compounds according to Formula I, wherein
either R³ and R⁴ taken together may form a ring selected from a group comprising a pyrrole ring, pyrroline ring, a pyrazole ring, pyrrolidine ring, dihydropyrimidine ring, a piperazine ring, an isoxazole ring, an isoxazolidine ring, an oxazolidine ring, wherein the ring may carry one or more substituents Y, or alternatively
- R³: may be H or CH₃ and

- R⁴: may be a pyrrole ring, pyrroline ring, a pyrazole ring, pyrrolidine ring, dihydropyrimidine ring, a piperazine ring, an isoxazole ring, an isoxazolidine ring, an oxazolidine ring,
wherein the ring may carry one or more substituents Y, wherein Y is as defined above.

In another preferred embodiment of the present invention, the compounds for treating diseases associated with fungal infection by a fungal species capable of forming biofilms are compounds according to Formula I, wherein
- R³: may be H or CH₃ and
- R⁴: may be a pyridazine ring, a pyrazine ring, pyrimidine ring, a dihydropyridazine ring, an isoxazoline ring, an oxazole ring, an oxazoline ring, a phenyl
wherein the ring may carry one or more substituents Y, wherein Y is as defined above.

In another preferred embodiment of the present invention, the compounds for treating diseases associated with fungal infection by a fungal species capable of forming biofilm are compounds according to Formula I, wherein
- R³: may be H or CH₃ and
- R⁴: is monoflourophenyl.

In a more preferred embodiment of the present invention, the compounds for treating diseases associated with fungal infection by a fungal species capable of forming biofilms are compounds selected from a group comprising:

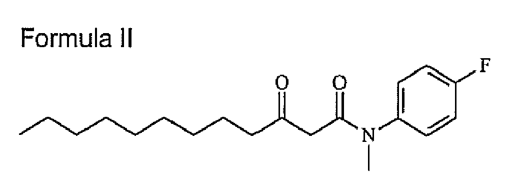

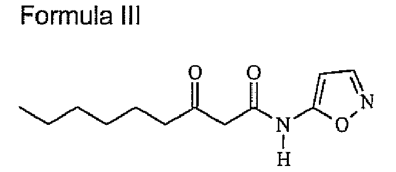

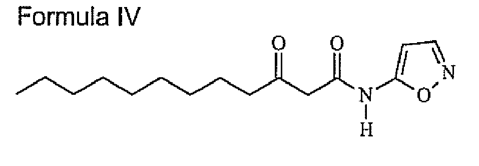

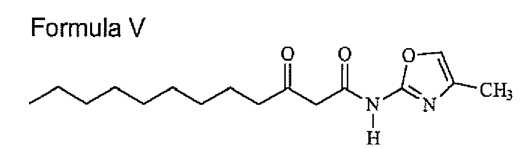

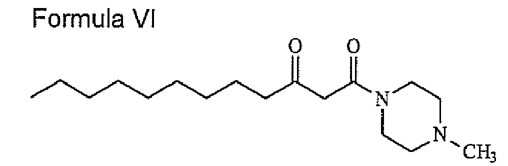

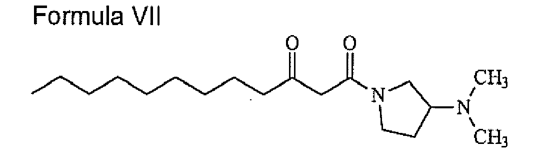

In a further preferred embodiment of the present invention, the compounds for treating diseases associated with fungal infection are compounds according to any of the previous formulae, wherein the fungal infection is associated with a fungal species capable of forming biofilms selected from the group comprising: *Candida, Cryptococcus, Histoplasma, Coccidioides, Paracoccidioides, Fusarium, Scopulariopsis, Blastomyces, Microsporum, Saccharomyces, Trichosporon, Geotrichum, Aspergillus, Penicillium, Sporothrix* and *Zygomyetes.*

In a further more preferred embodiment of the present invention, the compounds for treating diseases associated with fungal infection are compounds according to any of the previous formulae, wherein the fungal infection is associated with a fungal species capable of forming biofilms selected from the group comprising: candidiasis (general), cutaneous candidiasis, chronic cucocutaneous candidiasis, esophagitis, onychomycosis, oropharyngeal candidiasis, vulvovaginitis, invasive candidiasis including candida peritonitis, candida osteomyelitis, candida arthritis, CNS candidiasis, endophthalmitis, keratitis, biliary candidiasis, cardiac candidiasis, candida pneumonia, urinary candidiasis, hepatosplenic candidiasis, pancreatic candidiasis.

In another preferred embodiment of the present invention, the compounds according to any of the previous formulae are compounds for treating ophthalmological diseases associated with fungal infection by a fungal species capable of forming biofilms.

In another more preferred embodiment of the present invention, the compounds according to any of the previous formulae are compounds for treating mycotis keratitis associated with a fungal species capable of forming biofilms.

In another more preferred embodiment of the present invention, the compounds according to any of the previous formulae are compounds for treating endogenous oculomycosis associated with a fungal species capable of forming biofilms.

In another more preferred embodiment of the present invention, the compounds according to any of the previous formulae are compounds for treating extension oculomycosis associated with a fungal species capable of forming biofilms.

In another more preferred embodiment of the present invention, the compounds according to any of the previous formulae are compounds for treating contact lens-induced fungal infection by a fungal species capable of forming biofilms selected from a group comprising: microbial keratitis, acute red eye, contact lens-induced peripheral ulceration, infiltrative keratitis, and asymptomatic infiltrative keratitis.

A further embodiment of the present invention is the use of compounds according to any of the previous formulae to remove or diminish fungal infestation by a fungal species capable of forming biofilms in a non-therapeutic context selected from a group comprising: medical device products such as catheters, stents or implants, consumer care products such as home care products and cleaning solutions, food processing, agriculture, and water processing and treatment.

Another embodiment of the present invention is a method for treating a disease associated with fungal infection by a fungal species capable of forming biofilms comprising administering to a person in need thereof a compound according to Formula I, 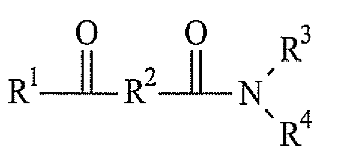 wherein

| | | |
|---|---|---|
| R¹ | represents | C₁-C₂₀-alkylene, or |
| | | C₁-C₂₀-alkenylene, or |
| | | C₁-C₂₀-alkynylene, |

which may contain one or more group(s) Z;
- Z: is selected from the group comprising S, O, N, NR₄, CO, CO₂, CS, SO or SO₂;

| | | |
|---|---|---|
| R² | represents | C₁-C₂₀-alkylene, or |
| | | C₁-C₂₀-alkenylene, or |
| | | C₁-C₂₀-alkynylene, |

- R³ and R⁴: taken together may form a 5-membered or 6-membered aromatic or non-aromatic ring which may contain one or more heteroatoms, wherein the heteroatoms may be independently of each other O or N;
wherein the ring may carry one or more substituents Y, or alternatively
- R³: may be H or CH₃ and
- R⁴: may be a 5-membered or 6-membered aromatic or non-aromatic ring which may contain one or more heteroatoms, wherein the heteroatoms may be independently of each other O or N;
wherein the ring may carry one or more substituents Y; and
- Y: is selected from the group comprising amine, alkylamine, C₁-C₃ alkyl, C₁-C₃ alkenyl, C₁-C₃ alkynyl, halogen, C₁-C₃ alkyl substituted by one or more halogens.

Another preferred embodiment of the present invention is a method for treating a disease associated with fungal infection by a fungal species capable of forming biofilm comprising administering to a person in need thereof a compound according to Formula I, wherein
- R¹: represents C₆-C₂₀-alkylene, or C₁-C₂₀-alkenylene, or C₁-C₂₀-alkynylene,

Another preferred embodiment of the present invention is a method for treating a disease associated with fungal infection by a fungal species capable of forming biofilms comprising administering to a person in need thereof a compound according to Formula I, wherein
- R¹: represents C₆-C₂₀-alkylene.

Another preferred embodiment of the present invention is a method for treating a disease associated with fungal infection by a fungal species capable of forming biofilms comprising administering to a person in need thereof a compound according to Formula I, wherein either R³ and R⁴ taken together may form a ring selected from a group comprising a pyrrole ring, pyrroline ring, a pyrazole ring, pyrrolidine ring, dihydropyrimidine ring, a piperazine ring, an isoxazole ring, an isoxazolidine ring, an oxazolidine ring, wherein the ring may carry one or more substituents Y, or alternatively
- R³: may be H or CH₃ and
- R⁴: may be a pyrrole ring, pyrroline ring, a pyrazole ring, pyrrolidine ring, dihydropyrimidine ring, a piperazine ring, an isoxazole ring, an isoxazolidine ring, an oxazolidine ring,
wherein the ring may carry one or more substituents Y, wherein Y is as defined above.

Another preferred embodiment of the present invention is a method for treating a disease associated with fungal infection by a fungal species capable of forming biofilms comprising administering to a person in need thereof a compound according to Formula I, wherein
- R³: may be H or CH₃ and
- R⁴: may be a pyridazine ring, a pyrazine ring, pyrimidine ring, a dihydropyridazine ring, an isoxazoline ring, an oxazole ring, an oxazoline ring, a phenyl
wherein the ring may carry one or more substituents Y, wherein Y is as defined above.

Another preferred embodiment of the present invention is a method for treating a disease associated with fungal infection by a fungal species capable of forming biofilms comprising administering to a person in need thereof a compound according to Formula I, wherein
- R³: may be H or CH₃ and
- R⁴: is monoflourophenyl,

Another more preferred embodiment of the present invention is a method for treating a disease associated with fungal infection by a fungal species capable of forming biofilms comprising administering to a person in need thereof one or more compounds selected from a group comprising:

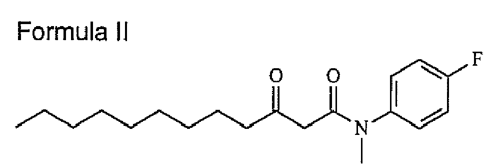

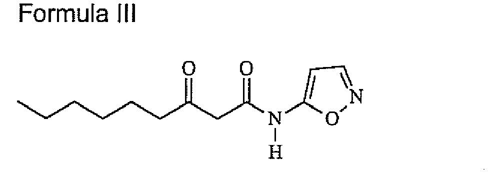

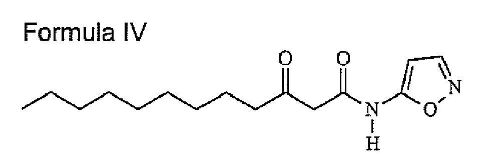

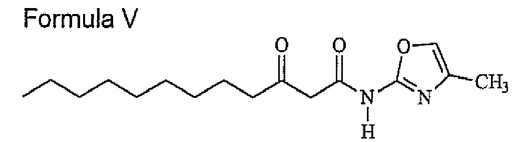

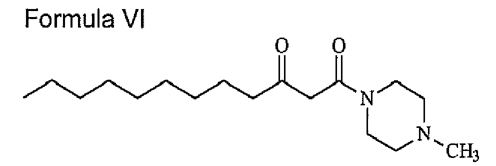

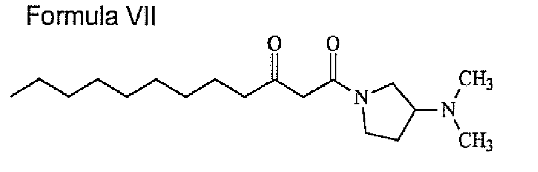

Another preferred embodiment of the present invention is a method for treating a disease associated with fungal infection by a fungal species capable of forming biofilms comprising administering to a person in need thereof a compound according to any of the previous formulae, wherein the fungal infection is associated with a fungal species by a fungal species capable of forming biofilms selected from the group comprising: *Candida, Cryptococcus, Histoplasma, Coccidioides, Paracoccidioides, Fusarium, Scopulariopsis, Blastomyces, Microsporum, Saccharomyces, Trichosporon, Geotrichum, Aspergillus, Penicillium, Sporothrix* and *Zygomyetes.*

Another more preferred embodiment of the present invention is a method for treating a disease associated with fungal infection by a fungal species capable of forming biofilms comprising administering to a person in need thereof a compound according to any of the previous formulae, wherein the fungal infection is associated with a fungal species by a fungal species capable of forming biofilms selected from the group comprising: candidiasis (general), cutaneous candidiasis, chronic cucocutaneous candidiasis, esophagitis, onychomycosis, oropharyngeal candidiasis, vulvovaginitis, invasive candidiasis including candida peritonitis, candida osteomyelitis, candida arthritis, CNS candidiasis, endophthalmitis, keratitis, biliary candidiasis, cardiac candidiasis, candida pneumonia, urinary candidiasis, hepatosplenic candidiasis, pancreatic candidiasis.

Another more preferred embodiment of the present invention is a method for treating mycotis keratitis associated with a fungal species capable of forming biofilms, comprising administering to a person in need thereof a compound according to any of the previous formulae.

Another more preferred embodiment of the present invention is a method for treating endogenous oculomycosis associated with a fungal species capable of forming biofilms ,comprising administering to a person in need thereof a compound according to any of the previous formulae.

Another more preferred embodiment of the present invention is a method for treating extension oculomycosis associated with a fungal species capable of forming biofilms, comprising administering to a person in need thereof a compound according to any of the previous formulae.

Another more preferred embodiment of the present invention is a method for treating contact lens-induced fungal infection by a fungal species capable of forming biofilms selected from a group comprising: microbial keratitis, acute red eye, contact lens-induced peripheral ulceration, infiltrative keratitis, and asymptomatic infiltrative keratitis, comprising administering to a person in need thereof a compound according to any of the previous formulae.

In the context of the present invention, fungi or fungal microorganism refer to in general to various types of fungi, such as moulds and yeast. For example, such fungi are, but are not limited to
*Candidae,* such as *Candida albicans, Candida tropicalis, Candida paratropicalis, Candida glabrata, Candida parapsilosis, Candida krusei, Candida lusitaniae, Candida kefyr, Candida guilliermondii, Candida dubliniensis, Candida famata, Candida inconspicua, Candida lambica and Candida lipolytica ;*
*Cryptococci, such as Cryptococcus neoformans, Cryptococcus albidus, Cryptococcus humicolus, Cryptococcus laurentii ;*
*Histoplasma capsulatum;*
*Coccidioides immitis;*
*Paracoccidioides brasiliensis;*
*Fusaria, such as Fusarium solani, Fusarium chlamydosporum, Fusarium dimerum, Fusarium moniliforme, Fusarium napiforme, Fusarium oxysporum, Fusarium proliferatum, Fusarium semitectum;*
*Scopulariopsis, such as Scopulariopsis acremonium, Scopulariopsis asperula, Scopulariopsis brevicaulis, Scopulariopsis brumptii, Scopulariopsis candidai, Scopulariopsis cinerea, Scopulariopsis paisii, Scopulariopsis trigonospora;*
*Blastomyces dermatitidis;*
*Microspora, such as Microsporum audouinii, Microsporum canis, Microsporum cookie, Microsporum distortum, Microsporum ferrugineum, Microsporum gallinae, Microsporum gypseum, Microsporum nanum, Microsporum vanbreusegh;*
*Saccharomycetes, such as Saccharomyces cerevisiae, Saccharomyces boullardii;*
*Trichospora, such as Trichosporon cutaneum, Trichosporon beigelii, Trichosporon asteroides, Trichosporon ovoides, Trichosporon inkin, Trichosporon asahii, and Trichosporon mucoides;*
*Geotricha, such as Geotrichum candidum, Geotrichum clavatum, Geotrichum fici;*
*Aspergilli, such as Aspergillus fumigatus, Aspergillus flavus, Aspergillus glaucus, Aspergillus granulosus, Aspergillus nidulans, Aspergillus niger, Aspergillus terreus, Aspergillus ustus, Aspergillus versicolor ;*
*Sporothrix, such as Sporothrix schenckii;*
*Penicillium marneffei;*
*Zygomyetes, such as Absidia, Apophysomyces, Cokeromyces, Cunninghamella, Mucor, Rhizomucor, Rhizopus, Saksenaea, Syncephalastrum*

Conditions which are within the scope of the present invention are exemplified in the following, but not limited to: Candidiasis (general), Cutaneous Candidiasis (burns, wounds), Chronic Mucocutaneous Candidiasis, Esophagitis, Onychomycosis, Oropharyngeal Candidiasis, Vulvovaginitis, invasive Candidiasis incl. Candida peritonitis, Candida Osteomyelitis, Candida Arthritis, CNS candidiasis, Endophthalmitis, Keratitis, Biliary Candidiasis, Cardiac Candidiasis, Candida Pneumonia, Urinary Candidiasis, Hepatosplenic Candidiasis, Pancreatic Candidiasis, Cryptococcosis, Meningitis, Meningoencephalitis, Histoplasmosis (general), disseminated infection with fever and weight loss, Coccidioidomycosis (general), Diffuse and focal pulmonary disease, Paracoccidioidomycosis (general), painful ulcerated lesions in the mouth, cutaneous lesions, lymphadenopathy, dysphagia, and hoarseness, Fusariosis (general), Keratitis, endophthalmitis, otitis media, onychomycosis, cutaneous infections particularly of burn wounds, mycetoma, sinusitis, pulmonary infections, endocarditis, peritonitis, central venous catheter infections, septic arthritis, disseminated infections, fungemia, onychomycosis especially of the toe nails, skin lesions, mycetoma, invasive sinusitis, keratitis, endophthalmitis, pulmonary infections, endocarditis, brain abscess, disseminated infections, Blastomycosis (general), localized pulmonary disease and disseminated infection, Dermatophytosis (infection in hair, skin or nails), Opportunistic infections in immunocompromised host incl. pneumonia, endocarditis, liver abscess, fungemia, and sepsis, Trichosporonosis (general), white piedra, superficial infections and invasive trichosporonosis, Geotrichosis (general), opportunistic infections in immunocompromised host incl. bronchial and pulmonary infections, Aspergillosis (general), allergic bronchopulmonary aspergillosis, Pulmonary aspergilloma, Invasive aspergillosis incl. Pulmonary aspergillosis, CNS aspergillosis, Sinonasal aspergillosis, Osteomyelitis, Endophthalmitis, Endocarditis, Renal abscesses, Cutaneous apergillosis (burns, post surgical wounds), Otomycosis, Exogenous endophthalmitis, Allergic fungal sinusitis, Urinary tract fungus balls, Sporotrichosis (general, "rose handler's disease"), Pulmonary and osteoarticular infections, granulamatous tenosynovitis and carpal tunnel syndrome, bursal infection, endophthalmitis, meningitis, invasive sinusitis, and disseminated sporotrichosis, Penicilliosis (general), Fever alone or with pulmonary infiltrates, lymphadenopathy, or cutaneous lesions, Zygomycosis incl. mucocutaneous and rhinocerebral infections, dialysis-associated peritonitis, renal infections, gastritis and pulmonary infections.

A number of pathological conditions are connected to fungal infestations. For instance, mycotic keratitis refers to the corneal infection associated with either filamentous fungi (moulds) or yeast. There are important epidemiological and clinical differences between these two forms. The most important risk factors related to fungal keratitis include trauma (generally with plant material), chronic ocular surface diseases, contact lens usage, surgery, corneal anesthetic abuse, and immunodeficiencies. Interestingly, fungal keratitis is a condition related to warm climates. In the southernmost states of the United States, fungal keratitis explains up to 35% of microbial keratitis cases compared with 1% in New York or Minnesota. The most common etiologic agents for this condition are *Fusarium spp, Aspergillus spp, Aspergillus flavus, Aspergillus fumigatus, Aspergillus niger, Acremonium, such as Acremonium* spp., *Candida albicans, Candida parapsilosis, Candida tropicalis, Bipolaris* spp., *Curvularia* spp., *Exserohilum* spp., *Fusarium oxysporum, Fusarium solani and Lasiodiplodia theobromae*. Endogenous oculomycosis is an eye infection which results from hematogenous dissemination of a systemic fungal disease. Eye involvement is typically a late event following widespread dissemination. The mycosis may involve the orbit, retina, optic nerve, sclera, conjunctiva, and adjacent tissue. The diseases in order of frequency are: candidiasis, cryptococcosis, coccidioidomycosis, blastomycosis, sporotrichosis, paracoccidioidomycosis, histoplasmosis, and aspergillosis. The most common of these forms of endogenous disease is candidal endophthlamitis. Extension oculomycosis represents a special form of rhinocerebral zygomycosis in the diabetic patient. The infection starts in the upper portion of the nasal septum and extends into the orbit of the eye, frontal sinuses, major cerebral vessels, and subsequently the central nervous system. *Rhizopus arrhizus* is the most common etiologic agent. Symptoms include orbital pain, ophthalomoplegia, localized anesthesia, proptosis, limitation of movement, fixation of the pupil, and loss of vision.

In a further embodiment, the compounds of the present invention and their pharmacologically acceptable salts can be administered directly to animals, preferably to mammals, and in particular to humans as antibiotics per se, as mixtures with one another or in the form of pharmaceutical preparations which allow enteral or parenteral use and which as active constituent contain an effective dose of at least one compound according to the present invention or a salt thereof, in addition to customary pharmaceutical excipients and additives. The compounds according to the present invention can also be administered in form of their salts, which are obtainable by reacting the respective compounds with physiologically acceptable acids and bases.

The therapeutics can be administered orally, e. g. in the form of pills, tablets, coated tablets, sugar coated tablets, lozenges, hard and soft gelatin capsules, solutions, syrups, emulsions or suspensions or as aerosol mixtures. Administration, however, can also be carried out rectally, e. g. , in the form of suppositories, or parenterally, e. g. in the form of injections or infusions, or percutaneously, e. g. in the form of ointments, creams or tinctures.

In addition to the active compounds according to the present invention, the pharmaceutical composition can contain further customary, usually inert carrier materials or excipients. Thus, the pharmaceutical preparations can also contain additives or adjuvants commonly used in galenic formulations, such as, e. g. fillers, extenders, disintegrants, binders, glidants, wetting agents, stabilizers, emulsifiers, preservatives, sweetening agents, colorants, flavorings or aromatizers, buffer substances, and furthermore solvents or solubilizers or agents for achieving a depot effect, as well as salts for modifying the osmotic pressure, coating agents or antioxidants. They can also contain two or more compounds of the Formula(I) or their pharmacologically acceptable salts and also other therapeutically active substances.

Thus, the compounds of the present invention can be used alone, in combination with other compounds of this invention or in combination with other active compounds, for example with active ingredients already known for the treatment of the afore mentioned diseases, whereby in the latter case a favorable additive effect is noticed. Suitable amounts to be administered to mammalian in particular humans range from 5 to 1000 mg.

To prepare the pharmaceutical preparations, pharmaceutically inert inorganic or organic excipients can be used. To prepare pills, tablets, coated tablets and hard gelatin capsules, e. g. lactose, corn starch or derivatives thereof, talc, stearic acid or its salts, etc. can be used. Excipients for soft gelatin capsules and suppositories are e. g. fats, waxes, semi-solid and liquid polyols, natural or hardened oils etc. Suitable excipients for the production of solutions and syrups are e. g. water, alcohol, sucrose, invert sugar, glucose, polyols etc.

Suitable excipients for the production of injection solutions are e. g. water, alcohol, glycerol, polyols or vegetable oils.

The dose can vary within wide limits and is to be suited to the individual conditions in each individual case. For the above uses the appropriate dosage will vary depending on the mode of administration, the particular condition to be treated and the effect desired. In general, however, satisfactory results are achieved at dosage rates of about 0,1 to 100mg/kg animal body weight preferably 1 to 50 mg/kg. Suitable dosage rates for larger mammals, e. g., humans, are of the order of from about 10 mg to 3 g/day, conveniently administered once, in divided doses 2 to 4 times a day, or in sustained release form.

In general, a daily dose of approximately 0,1 mg to 5000 mg, preferably 10 to 500 mg, per mammalian in particular human individual is appropriate in the case of the oral administration which is the preferred form of administration according to the invention. In the case of other administration forms too, the daily dose is in similar ranges. The compounds of Formula (I) can also be used in the form of a precursor (prodrug) or a suitably modified form, that releases the active compound in vivo.

In a further embodiment, the compounds of the present invention can be used as pharmacologically active components or ingredients of medical devices, instruments and articles with an effective dose of at least one compound according to the present invention or a salt thereof. The amount of the compounds used to coat for example medical device surfaces varies to some extent with the coating method and the application field. In general, however, the concentration range from about 0,01 mg/cm² to about 100 mg/cm². In a similar way the amount of the compounds has to be adjusted to the application mode if the compounds of the invention are used as components or ingredients in cleaning or treatment solutions. In general, effective dosages range from about 0,1, µM to about 1000 mM.

The compounds of the present invention can be synthesized as disclosed in EP 1 478 364.

### EXAMPLES

### TEST METHOD: MBEC^{™} Assay

The compound to be tested was dissolved in DMSO to obtain a concentration that was 100 times that of the final concentration required (1mM for the 10µM assay and 10mM for the 100µM assay). 1.5 µL of the above solution was placed into each well of a 96 well plate (final volume in each well = 150 µL, final DMSO concentration of 1% (v/v)).

To grow the organism and form a biofilm, a cryogenic stock of *Candida paratropicalis* (at - 70°C) was used. A first sub-culture was streaked out onto TSA (tryptic soy agar), upon which the plate was incubated at 37±2°C for 24 hours and stored wrapped in parafilm at 4 °C afterwards. From this first sub-culture, a second sub-culture was streaked out onto TSA. The plate was incubated at 37±2°C for 24 hours. The second sub-culture was used within 24 hours starting from the time it was first removed from incubation. Using the second sub-culture an inoculum in 3 mL sterile water that matches a 0.5 McFarland Standard (1.5 x 10⁸ cells per mL) was created in a glass test tube using a sterile cotton swab. 2.2 mL of this solution was diluted in 22mL TSB (tryptic soy broth) medium to a cell density of approximately 6.0 x 10⁵ cells per mL. The diluted organism was inverted 3-5 times to achieve uniform mixing of the organism. One sample (100µl) of the diluted organism was used for an inoculum check by serially diluting and spot plating on TSA.
To prepare a challenge plate, 20 µl of the diluted organism was added to each well of a 96 well plate except the sterility control wells. The test compounds were added to the wells to make up final concentrations of 10 µM and 100 µM (1.5 µL /well) in the designated location in the test plate. 128.5 µL of TSB was placed in each of the wells. 130 µL of media was added to the growth control wells. 128.5 µL of media+ 1.5 µL of DMSO were added to a second set of growth control wells. 150 µL of media was added to the sterility controls. Each sample was run in triplicate. The plate was covered by a lid with a peg per well and wrapped in parafilm and placed on a shaker in a humidified incubator (GeneVac) at 37±2°C for 24 hours set at 110 rpm.
Rinse plate(s) of 0.9% saline (200 µL per well) were prepared in a sterile 96 well microtitre plate. Lid-associated pegs were rinsed in 0.9% saline for approximately 1-2 minutes. The peg lid was transferred to the recovery media (1.0% Tween 80 in CAMHB) then sonicated for 30 minutes to dislodge surviving biofilm. Following sonication, 100 µl from each well of the 96 well plate was placed into the first 12 wells of the first row of a 96 well microtiter plate. 180 µl of 0.9% sterile saline was placed in the remaining rows. A serial dilution (10⁰-10⁻⁷) was prepared by moving 20 µl down each of the 8 rows. 20µl from each well was removed and spot plated on a prepared TSA. In the remaining recovery plate, 100 µl of fresh growth media (1.0% Tween 80 in CAMHB) was placed in each well to replace the amount removed. The recovery plate was covered with a regular lid and incubated at 37±2°C for 24 hours and read visually to confirm antimicrobial activity of the test compounds.

The following compounds have been tested regarding their efficiency (logR = log reduction) against *Candida paratropicalis:*

| Formula | Example No. | Biofilm inhibition 10 µM (logR) | Biofilm inhibition 100 µm (logR) |
|---|---|---|---|
| 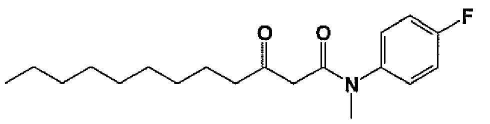 | 1 | - | 0.62 |
| 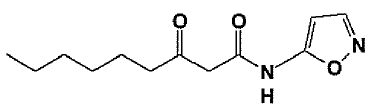 | 2 | 1.12 | 0.65 |
| 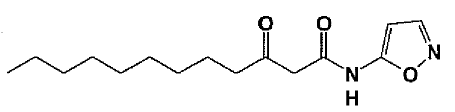 | 3 | 1.26 | 1.10 |
| 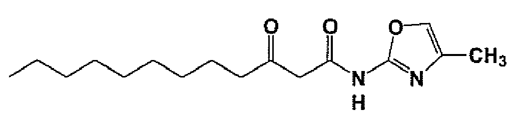 | 4 | - | 1.71 |
| 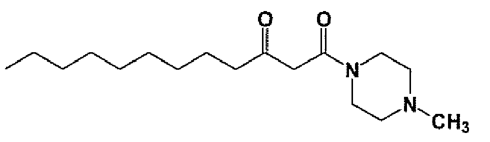 | 5 | - | 2.83 |
| 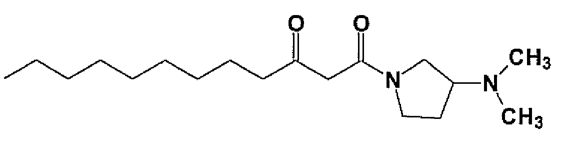 | 6 | - | 3.87 |

## Claims

1. Compounds according to Formula I for treating diseases associated with fungal infection by a fungal species capable of forming biofilms, 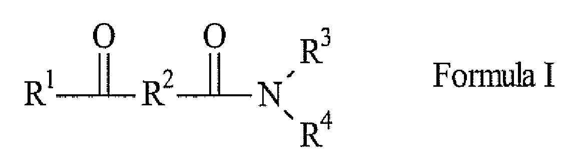 wherein
| | | |
|---|---|---|
| R¹ | represents | C₁-C₂₀-alkylene, or |
| | | C₁-C₂₀-alkenylene, or |
| | | C₁-C₂₀-alkynylene, |
which may contain one or more group(s) Z;
Z is selected from the group comprising S, O, N, NR₄, CO, CO₂, CS, SO or SO₂;
| | | |
|---|---|---|
| R² | represents | C₁-C₂₀-alkylene, or |
| | | C₁-C₂₀-alkenylene, or |
| | | C₁-C₂₀-alkynylene, |
R³ and R⁴ taken together may form a 5-membered or 6-membered aromatic or non-aromatic ring which may contain one or more heteroatoms, wherein the heteroatoms may be independently of each other O or N;
wherein the ring may carry one or more substituents Y, or alternatively
R³ may be H or CH₃ and
R⁴ may be a 5-membered or 6-membered aromatic or non-aromatic ring which may contain one or more heteroatoms, wherein the heteroatoms may be independently of each other O or N;
wherein the ring may carry one or more substituents Y; and
Y is selected from the group comprising amine, alkylamine, C₁-C₃ alkyl, C₁-C₃ alkenyl, C₁-C₃ alkynyl, halogen, C₁-C₃ alkyl substituted by one or more halogens.

2. Compounds according to claim 1 for treating diseases associated with fungal infection by a fungal species capable of forming biofilms,
wherein
R¹ represents C₆-C₂₀-alkylene, or C₁-C₂₀-alkenylene, or C₁-C₂₀-alkynylene,

3. Compounds according to claim 1 or 2 for treating diseases associated with fungal infection,
wherein
R¹ represents C₆-C₂₀-alkylene.

4. Compounds according to claims 1 to 3 for treating diseases associated with fungal infection by a fungal species capable of forming biofilms,
wherein
either R³ and R⁴ taken together may form a ring selected from a group comprising a pyrrole ring, pyrroline ring, a pyrazole ring, pyrrolidine ring, dihydropyrimidine ring, a piperazine ring, an isoxazole ring, an isoxazolidine ring, an oxazolidine ring, wherein the ring may carry one or more substituents Y, or alternatively
R³ may be H or CH₃ and
R⁴ may be a pyrrole ring, pyrroline ring, a pyrazole ring, pyrrolidine ring, dihydropyrimidine ring, a piperazine ring, an isoxazole ring, an isoxazolidine ring, an oxazolidine ring,
wherein the ring may carry one or more substituents Y, wherein Y is as defined in claim 1.

5. Compounds according to claims 1 to 3 for treating diseases associated with fungal infection by a fungal species capable of forming biofilms,
wherein
R³ may be H or CH₃ and
R⁴ may be a pyridazine ring, a pyrazine ring, pyrimidine ring, a dihydropyridazine ring, an isoxazoline ring, an oxazole ring, an oxazoline ring, a phenyl
wherein the ring may carry one or more substituents Y, wherein Y is as defined in claim 1.

6. Compounds according to claims 1 to 3 for treating diseases associated with fungal infection by a fungal species capable of forming biofilms,
wherein
R³ may be H or CH₃ and
R⁴ is monoflourophenyl,

7. Compounds according to claim 1 to 3 for treating diseases associated with fungal infection by a fungal species capable of forming biofilms, wherein the compound is selected from a group comprising:

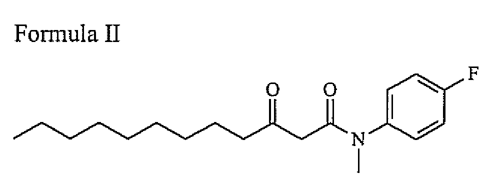

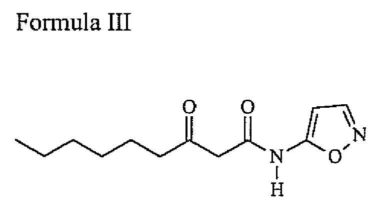

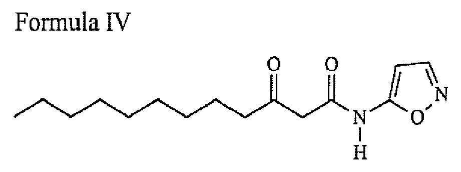

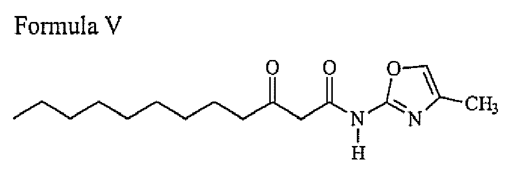

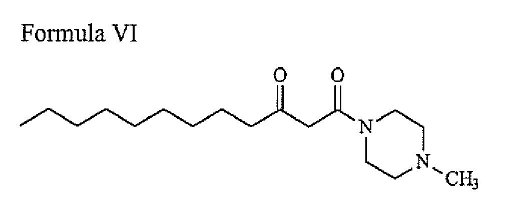

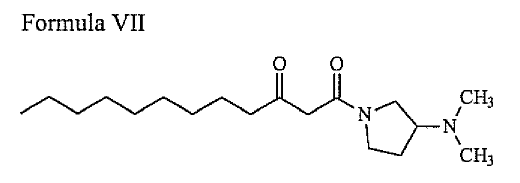

8. Compounds according to claims 1 to 7 for treating diseases associated with fungal infection by a fungal species capable of forming biofilms,
wherein this fungal infection is associated with a fungal species selected from the group comprising: *Candida, Cryptococcus, Histoplasma, Coccidioides, Paracoccidioides, Fusarium, Scopulariopsis, Blastomyces, Microsporum, Saccharomyces, Trichosporon, Geotrichum, Aspergillus, Penicillium, Sporothrix* and *Zygomyetes.*

9. Compounds according to claims 1 to 7 for treating a disease associated with a fungal species capable of forming biofilms selected from a group comprising: candidiasis (general), cutaneous candidiasis, chronic cucocutaneous candidiasis, esophagitis, onychomycosis, oropharyngeal candidiasis, vulvovaginitis, invasive candidiasis including candida peritonitis, candida osteomyelitis, candida arthritis, CNS candidiasis, endophthalmitis, keratitis, biliary candidiasis, cardiac candidiasis, candida pneumonia, urinary candidiasis, hepatosplenic candidiasis, pancreatic candidiasis.

10. Compounds according to claims 1 to 7 for treating ophthalmological diseases associated with fungal infection by a fungal species capable of forming biofilms.

11. Compounds according to claims 1 to 7 for treating mycotis keratitis associated with a fungal species capable of forming biofilms.

12. Compounds according to claims 1 to 7 for treating endogenous oculomycosis associated with a fungal species capable of forming biofilms.

13. Compounds according to claims 1 to 7 for treating extension oculomycosis associated with a fungal species capable of forming biofilms.

14. Compounds according to claims 1 to 7 for treating contact lens-induced fungal infection by a fungal species capable of forming biofilms selected from a group comprising: microbial keratitis, acute red eye, contact lens-induced peripheral ulceration, infiltrative keratitis, and asymptomatic infiltrative keratitis.

15. Use of a compound according to any of claims 1 to 7 to remove or diminish fungal infestation associated with a fungal species capable of forming biofilms in a non-therapeutic context.
